# EUROPEAN PATENT APPLICATION

(11) **EP 1 785 088 A1**
(43) Date of publication of application: **16.05.2007**
(21) Application number: 05110708.4
(22) Date of filing: 14.11.2005
(51) Int. Cl.: A61B 5/022

(54) **A system and method for the management and control of cardiovascular related diseases, such as hypertension**

(71) Applicant: Congener Wellness Corp., Nei-Hu Taipei 114 (TW)
(72) Inventor: Lin, Kin-Yuan, Taipei, Taiwan (CN); Yen, Chung-Yueh, 104, Taipei City, Taiwan (CN)
(74) Representative: Müller, Christoph Emanuel

(57) **Abstract**

A system and a method for the management or control of cardiovascular related diseases, in particular of hypertension, basically comprises a blood pressure measuring device and means for determining an activity status of the autonomic nervous system. In particular, this status is determined on the basis of heart rate variability or on the basis of a composition ratio of granulocytes and lymphocytes in leukocytes of the individual.

## Description

The invention relates to a system and to a method for the management or control of cardiovascular related diseases, in particular hypertension according to the independent patent claims.

It is widely accepted that hypertension and other cardiovascular diseases is one the major problem in health systems of most countries. Hypertension is currently treated in particular by prescribing medication such as alpha-blockers, beta-blockers, calcium channel-blockers, diuretic, angiotensin converting enzyme inhibitors or angiotensin II receptor blockers. However, such medication is often made on a try and error basis. Consequently, many patients are prescribed with unnecessary medication or suffer from side effects.

According to recommendations of the British Hypertension Society there are suggested some combinations for blood pressure lowering drugs following the so called AB/CD rule (see e.g. Gordon T. McInnes, "Lowering blood pressure for cardiovascular risk reduction", Journal of Hypertension 2005, 23 (suppl 1): S. 3 to S. 8, with reference to Brown et al., "Better blood pressure control: how to combine drugs", J Hum Hypertens 2003; 17: 81-86). While such a combination of medication may lead to a better and more focused use of medication, there are still drawbacks in context with such suggestions.

In "2003 European Society of Hypertension - European Society of Cardiology Guidelines for the Management of Arterial Hypertension" (Journal of Hypertension 2003, 21: 1011 - 1053)there are disclosed a variety of different causes of hypertension and of possible combinations of different anti-hypertensive medication.

Furthermore, other treatments such as lifestyle change are disclosed.

Samual J. Mann ("Healing Hypertension, A Revolutionary New Approach", Hypertension Center of the New York Presbyterian Hospital-Cornell Medical Center) has made a differentiation between different organs as driving factors for hypertension, in particular the brain or the kidneys. According to Mann, it would be logical to select drugs this way on the basis of the driving factor of hypertension. However, neither renin measurement nor any other biochemical test can distinguish brain driven from kidney driven hypertension.

It is thus an object of the present invention to overcome the drawbacks of the prior art, in particular to provide a system and a method for the management or control of cardiovascular related diseases such as hypertension which allows to provide an individual diagnosis and an individual suggestion for treatment of a patient in particular in view of hypertension. In particular, the method and the system according to the invention shall help to reduce or avoid medication errors and limit the number of medication prescribed to a particular individual to the maximum extent possible and to reduce side effects and to improve compliance. In particular the method and the system according to the invention provide a proactive and side effect free complementary and alternative method of treatment to the individual, who are in the pre-hypertension stage or medium or low risk level where the drug treatment is not appropriate but/and life style change can not be implemented in short term.

According to Possidente ("The Role of Home Blood Pressure Monitoring in Hypertension Control", Judy Possidente Kaufman, Journal of Clinical Hypertension 3 (3): 171 - 173, 2001) home monitoring supports physicians or other health care professionals in caring for patients with hypertension by providing plenty of data. Kaufman sees one major problem in hypertension management in sub optimal adherence to blood pressure lowering medications. In particular, because a majority of hypertensive patients require more than one medication, it is assumed that complex medication regimes contribute to poor treatment adherence. According to Kaufman, an integrated comprehensive approach of hypertension and its management is often lacking.

These and other objects are solved with a system and a method according to the independent patent claims.

The system and the method according to the invention generally are helpful in context with cardiovascular related diseases. In particular, the invention is directed to hypertension. However, the invention is not limited to hypertension but can be equally applied in context with other cardiovascular related diseases. In particular, diabetes, metabolic syndrome or dyslipidaemia may be managed and related measurement values such as glucose, insulin or cholesterol level might be considered. These diseases and symptoms are in close interrelationship in view of their effect on cardiovascular diseases and hypertension. According to the present invention, one major focus is hypertension. However, other indications or symptoms may be taken into consideration.

The system for the management of hypertension of an individual according to the invention comprises a blood pressure measuring device for measuring the blood pressure of the individual. In addition, the system comprises means for detecting the activity of the autonomic nervous system. Autonomic nervous system activity status may be an indication of the root cause for hypertension as will be shown herein after. According to a first embodiment of the invention, the system may comprise a heart rate variability detection means for determining the heart rate variability of the individual. Preferably, the system then comprises a calculation means for determining the autonomic nervous system activity status based on the heart rate variability. The system may also display heart rate variability information without further calculation. In this case, the system nevertheless has means for determination of the autonomic nervous system activity status.
It is known that the heart rate variability is a tool to assess the autonomic function (see e.g. J. P. Singh et al., "Reduced Heart Rate Variability and New-Onset Hypertension, Insights Into Pathogenesis of Hypertension: The Framingham Heart Study, in Hypertension, 1998;32:293-297).

Heart rate variability (HRV) is a useful noninvasive tool to assess cardiac autonomic function.

HRV has considerable potential to assess the role of autonomic nervous system fluctuations in normal healthy individuals and in patients with various cardiovascular and noncardiovascular disorders. HRV studies should enhance our understanding of physiological phenomena, the actions of medications, and disease mechanisms. (see "Heart Rate Variability: Standards of Measurement, Physiological Interpretation, and Clinical Use", Circulation 93 (5): 1043).

Therein, different measurement methods for determining heart rate variability are disclosed.

According to an alternative embodiment of the invention, the activity of the autonomic nervous system is determined on the basis of the composition ratio of granulocytes and lymphocytes in leukocytes. This ratio also may be an indication for the autonomic nervous system activity status based on the findings of Toru Abo in "Immunomodulation by the autonomic nervous system", Recent Res.Devel.Immunology, 4 (2002), page 559-578.

According to the present invention, there is provided a complete system which at the same time allows to acquire blood pressure data of an individual and autonomic nervous system activity status, e.g. by determining heart rate variability data of the individual. If the blood pressure measuring device indicates that the individual suffers or may suffer from hypertension, the calculation means may determine an activity status of the autonomic nervous system on the basis of the heart rate variability or on the basis of a composition ratio of granulocyte and lymphocyte in leukocytes. In particular, an activity status of the sympathetic nervous system or of the parasympathetic nervous system will be determined.

According to a preferred embodiment of the invention, the system further comprises a display for display of the autonomic nervous system activity status or of a composition ratio of granulocytes and lymphocytes in leukocytes. The display of this status allows a doctor or care person to decide whether hypertension rather may be kidney driven or brain driven. Depending on this assessment, a specific prescription for medication may be made. It is, however, also possible not to directly display the autonomic nervous system activity status but to base further calculations on this status and to display further information as will be shown herein after.

According to a preferred embodiment of the invention, the blood pressure measuring device and the heart rate variability determination means are integrated within the same physical device.

Such a device allows for very easy measurements. One single device will allow the user or the care person to gather blood pressure data and heart rate variability data as well as to determine the driving factor for possible hypertension.

According to a first embodiment of the invention, the heart rate variability determination means may be adapted to determine heart rate variability of the basis of pressure pulse measurement. This is particularly simple if such a measurement device is integrated with a blood pressure monitor for automatically measuring the blood pressure. Such blood pressure monitors determine pressure pulse data. No further measuring sensors are thus necessary. It is sufficient to provide the blood pressure measuring device with further calculating arrangements for determining the heart rate variability based on pressure pulse data.

Alternatively, it is, however, also possible to include an ECG measuring unit within the heart rate variability determination means. In this case, the heart rate variability determination means are adapted to determine heart rate variability on the basis of an electrocardiogram of the individual.

The system according to the present invention preferably may comprise a user input interface adapted for entry of information relating to at least one risk factor of the individual to suffer from diseases, in particular from cardiovascular diseases or adapted for entry of influencing or driving factors relating to such a disease. Such a system allows to display information in view of an optimum treatment of the patient on the basis of measurements done by the system and of risk factors or driving factors entered into the system. A device allowing entry of such data is e.g. shown in EP 1 297 783 A1.

In particular, the risk factors may be factors selected from the group of age and sex, total cholesterol level, smoking, family history of cardiovascular disease, diabetes, target organ damage or associated clinical conditions. The driving factor also may include information relating to the level of renin, insulin and lipoid profile or genetic test information. Low renin may indicate that the individual rather suffers from salt sensitive hypertension. Especially in combination with the nervous autonomic system activity status, this allows to make a differentiation between brain and kidney driven hypertensive individuals. Other biochemistry data which are relevant in context with cardiovascular diseases may also be entered.

According to a further preferred embodiment of the invention, the blood pressure measuring device is designed for measurement of blood pressure according to a predetermined or predeterminable clinically validated measurement schedule. Such a device has been disclosed in the co-pending application PCT/EP2005/0502739.

Such a clinically validated blood pressure monitor allows for home blood pressure measurement. In view of costs home blood pressure measurements are preferred. Because of the so called white coat effect, home blood pressure measurements with an appropriate tool also lead to more reliable results.

According to the further preferred aspect of the invention, the system may comprise a decision making means. This decision making means may be coupled or adapted to be coupled to the blood pressure measuring device and to the heart rate variability determination means. The decision making means comprises data processing means for determining a risk level for the patient to suffer from a disease and/or for determining the causes for such a potential disease and/or for determining appropriate measures for prevention or treatment of said patient in view of this disease. Such a decision making means makes the system an integrated complete system for making measurements and for giving treatment of medication suggestions.

Such a data processing means is particularly adapted to make medication suggestions based on the entered and/or acquired data. In particular, medication or treatment suggestions may be made based on the finding of the driving factor of hypertension, in particular whether hypertension is brain driven or kidney driven.

The system may comprise display means for display of at least one of short, medium or long term measures in view of treatment of risk factors or a disease.

The decision making means may be a separate device and may be provided with communication interfaces, e.g. wireless communication for communication with the blood pressure measuring device or the heart rate variability determination means. It is, however, also possible to integrate the decision making device in the same physical device as the blood pressure measuring device or the heart rate variability determination means. A separate decision making device has, however, certain advantages: in particular, it is possible to have one decision making device located at the premises of a care person. Patients may make their home measurements and bring their measuring device to the care person where data are exchanged with the decision making device through the communication interface. It is therefore not necessary for each individual using the system according to the present invention to purchase such decision making device.

According to a further preferred aspect of the invention, the system also may comprise follow-up means. Such follow-up means can be in particular means for following compliance of the patient with a specific medication or treatment schedule. Such means for following compliance are known in the art. In combination with the system according to the invention, such follow-up or compliance means are particularly useful.

The method according to the present invention is used for operation of a system for management of hypertension of an individual. In particular, the method is used for operation of a system as disclosed herein above. In a first step, blood pressure data of the individual are acquired. In a further step, the activity of the autonomic nervous system is determined as a activity status. This may be done on the basis of heart rate variability data of the individual. Based on these heart rate variability data, an autonomic nervous system activity status is determined. Alternatively, the autonomic nervous system activity status may also be determined on a composition ratio between granulocytes and lymphocytes in leukocytes of the individual. In a final step, reasons for hypertension are determined, risk levels are stratified and/or appropriate long, medium or short term measures are selected on the basis of the blood pressure data and the autonomic nervous system activity status.

According to a preferred embodiment of the invention, the autonomic nervous system activity status is displayed on a display.

The heart rate variability may be determined on the basis of pressure pulse information measured by a blood pressure monitor or on the basis of electrocardiographic information measured by an ECG device.

According to a further preferred step, information relating to risk factors or driving factors for hypertension may be acquired.

It is further preferred to measure the blood pressure according to a clinically validated measurement schedule.

According to the method, it is possible to determine specific measures to be taken in view of prevention or treatment of hypertension or associated diseases. Such measures may also be displayed.

While this invention has been shown with respect to driving causes for a treatment of hypertension, a similar system and method can be used for treatment of other diseases, in particular for any kind of metabolic syndrome.

The invention will now be explained in more detail with reference to the embodiments and the accompanying drawings which show:
- Figure 1:: A schematic representation of a first embodiment of a device according to the invention
- Figure 2:: A flow chart of operation of the device according to Figure 1
- Figure 3:: A schematic representation of a device of a second embodiment of the invention
- Figure 4:: A flow chart showing the operation of the device according to Figure 3
- Figure 5:: A schematic representation of a device according to a third embodiment of the invention
- Figure 6:: A schematic representation of a device according to a fourth embodiment of the invention
- Figure 7:: A flow chart of the operation of the devices according to Figure 5 and 6
- Figure 8:: A schematic representation of a fifth embodiment of the invention
- Figure 9:: A schematic representation of a sixth embodiment of the invention
- Figures 10a to 10g:: Flow charts for operation of the devices of Figure 8 and 9
- Figure 11:: A schematic representation of a seventh embodiment of the invention
- Figure 12:: A schematic representation of a eighth embodiment of the invention
- Figure 13a and 13b:: A flow chart for operation of the devices according to Figure 11 and 12
- Figure 14:: A table showing risk stratification

- Figure 15:: A flow chart showing a procedure for determining heart rate variability
- Figure 16a to 16d:: Several graphics in relation to determination of heart rate variability and
- Figure 17:: A block diagram showing processes and causes leading to hypertension and other cardiovascular related diseases
- Figure 18, 19:: A schematic representation of a device according to a 9^{th} and 10^{th} embodiment of the invention
- Figure 20:: A flow chart of the operation of the devices according to Figures 18 and 19.

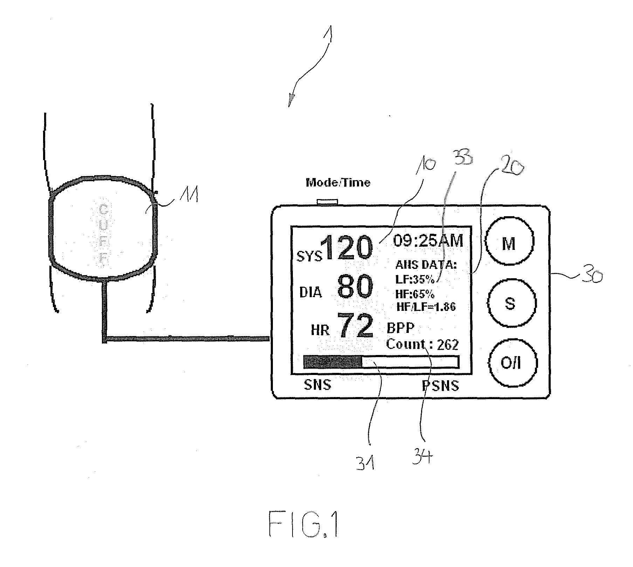

Figure 1 shows a first embodiment of a system 1 according to the present invention. The system 1 includes a blood pressure measuring device 10 for measuring pressure pulses in a cuff 11 and for determining systolic and diastolic blood pressure values as well as a heart rate. The system 1 is further provided with heart rate variability detection means 20 (not shown in detail). The heart rate variability detection means 20 determines heart rate variability on the basis of blood pressure pulse measurements. The heart rate variability determination means 20 and the blood pressure measuring device are integrated within a housing 30. The heart rate variability determination means uses pressure signals from the cuff for calculation of the heart rate variability. Calculation is done in a microprocessor of the blood pressure measuring device which is provided with a specific program for this purpose. The device is further provided with a heart rate variability display 33. In this display 33, low frequency LF and high frequency HF percentages and a high to low frequency ratio HF/LF are displayed.

In a blood pressure pulse display 34 the number of blood pressure pulses considered for determination of the heart rate variability is displayed.

Based on the ratio HF/LF between high frequency and low frequency percentage, an autonomic nervous system activity status is displayed as a bar graph in an activity status display 31.

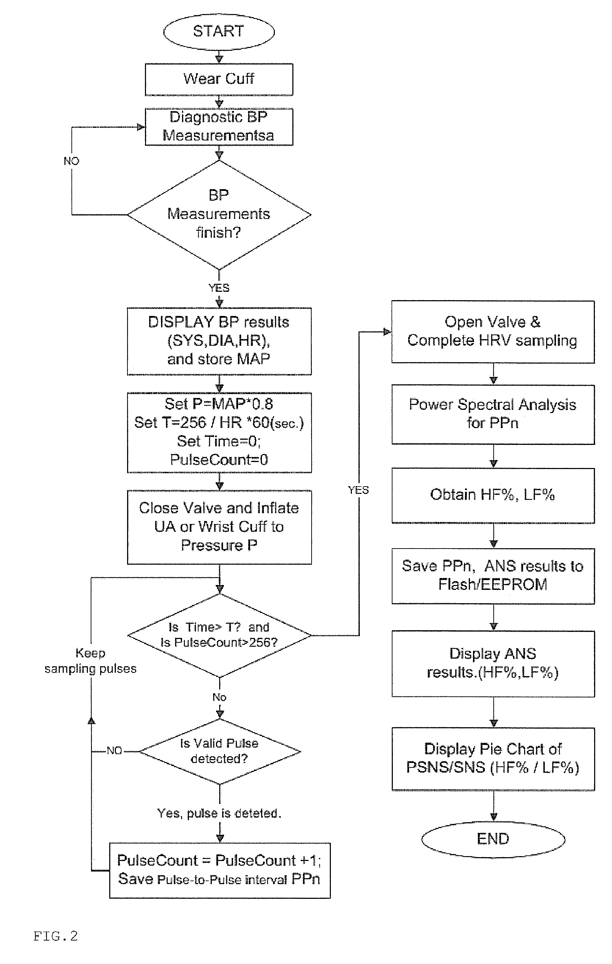

The operation of the device of Figure 1 is shown in the flow chart of Figure 2. Once the cuff has been properly placed around a patient's upper arm or wrist, blood pressure measurements are started. Blood pressure measurements are made according to a diagnostically or clinically validated measurement schedule. This allows for reliable home blood pressure measurements. Once the measurements are finished, the blood pressure results, in particular the systolic SYS and the diastolic DIA pressure and the heart rate HR are displayed. The mean arterial pressure MAP is stored. Determination of these values is made in accordance with known methods, in particular the oscillometric method.

In a next step, heart rate variability measurements are made. Before starting heart rate variability measurement, a plurality of parameters are set. The cuff pressure P will be set at about 80% of the mean arterial pressure MAP which has been previously determined. The measurement time T will be determined in dependence of the heart rate HR. The duration of the measurement shall be such that approximately 256 pulses will be considered. Before start of the heart rate variability measurement, variables Set Time and PulseCount are set to zero.

In the next step, the valve of the cuff is closed and the cuff, which can be placed on the upper arm or on the wrist is inflated to the pressure P which has been previously set to 80% of the mean arterial pressure MAP. As long as there are less than 256 pulses and the time is below the previously set measurement time T, the pulses are detected and pulse intervals PPN are saved. Each time a pulse interval is saved, the variable PulseCount is increased by 1. If no valid pulse is detected, no pulse time intervals are stored and the variable PulseCount is not increased. Sampling of pulses is continued in this case.

As soon as the measurement time T has been reached and the pulse count variable is above 256, the valve of the cuff is opened such as to release pressure. The heart rate variability sampling is completed.

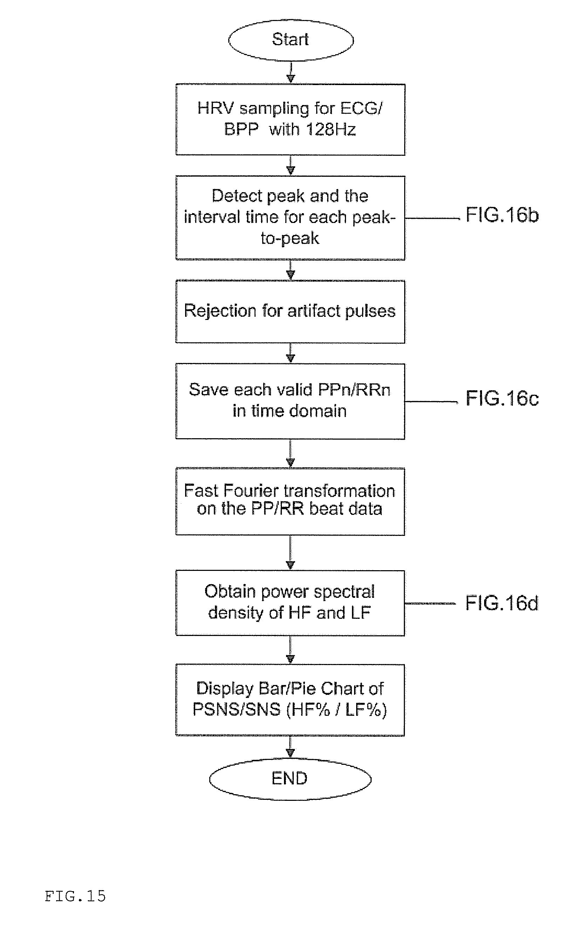

In a next step, a power spectral analysis of the pulse time difference PPN is made. During this analysis, high frequency percentages and low frequency percentages are determined. Determination of the heart rate variability will be explained in more detail with reference to Figures 15 and 16a to 16e.

The pulse to pulse intervals PPN and the autonomic nervous system results ANS subsequently are stored in a memory such as a flash or a EEPROM. The autonomic nervous system results ANS are displayed as a high frequency and a low frequency percentage in the display 33 as shown in Figure 1. A graphic bar representation of the parasympathetic nervous system activity and the sympathetic nervous system activity is displayed on the display 31 as a ratio between the high frequency percentage and the low frequency percentage.

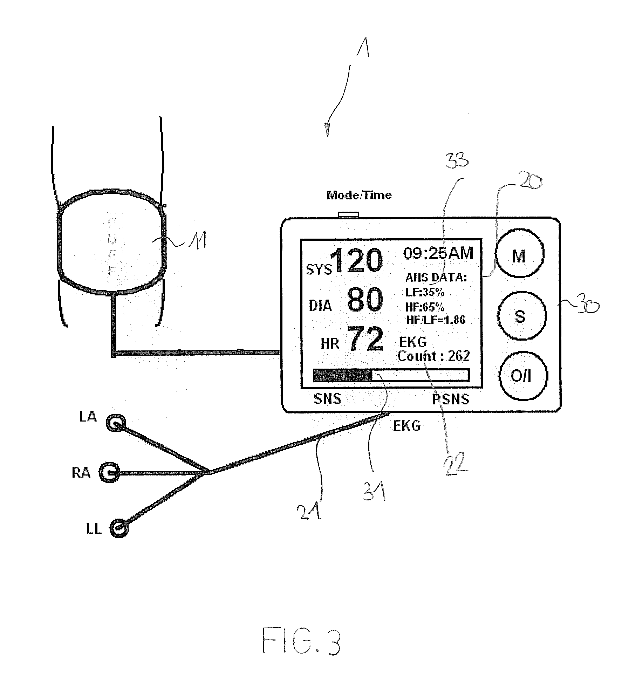

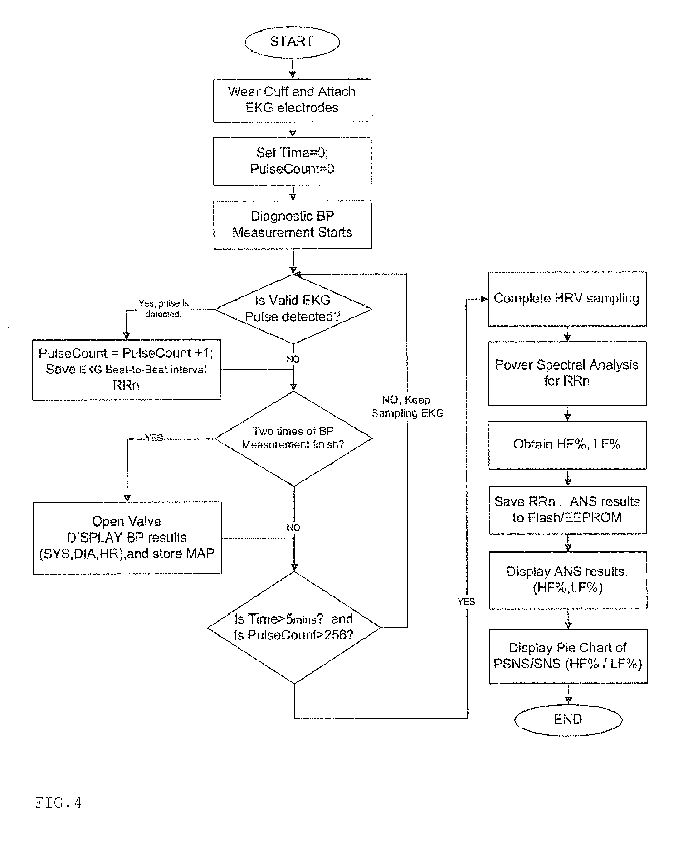

Figure 3 shows a second embodiment of the invention. The embodiment according to Figure 3 is substantially identical to the embodiment of Figure 1. However, heart rate variability is not measured on the basis of blood pressure pulses but rather on the basis of electrocardiographic information which are acquired with a electrocardiographic measuring unit 21. The ECG measuring unit 21 includes electrodes for acquiring an electrocardiogram in a known manner. Instead of a blood pressure pulse display, an electrocardiographic pulse count 22 is provided where the number of pulses considered during heart rate variability analysis will be displayed. Operation of the device as shown in Figure 3 will be explained with reference to the flow chart shown in Figure 4.

Contrary to the operation of the system of Figure 1, according to Figure 4, electrocardiographic information may be detected in parallel to blood pressure measurement. During the analysis, two blood pressure measurements are made. Once two measurements have been made, the valve of the cuff are opened and blood pressure results are displayed. Sampling of electrocardiographic data is continued until the measurement time T is above 5 minutes and the number of counted pulses PulseCount is above 256. Because the electrocardiographic sampling and blood pressure measurement is started at the same time, it is not possible to determine a measurement time on the basis of the heart rate in advance. Therefore, the measurement time is set equal to 5 min./300 sec.

The preferred number of sampled pulses is 256. However, to prevent too long measurement times, a measurement time will be also set.

Completion of the heart rate variability sampling and determination of autonomic nervous system data ANS is done in the same manner as explained with reference to Figure 2.

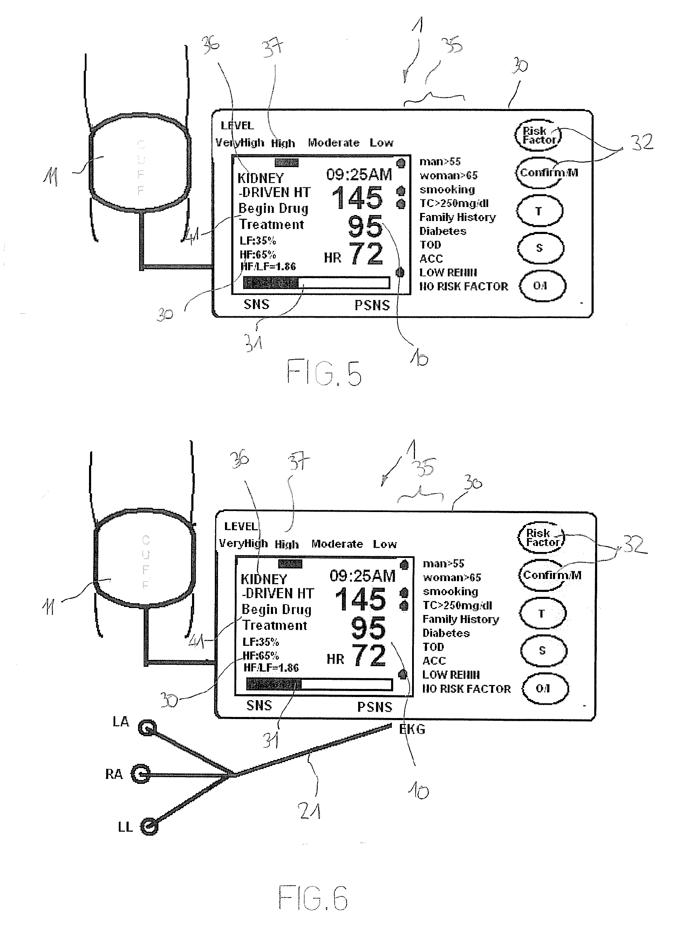

Figure 5 shows a third embodiment of the invention. The system 1 is substantially identical to the system as shown in Figure 1. In addition to the device shown in Figure 1, the device 1 shown in Figure 5 comprises a user input interface 32 for entry of information relating to risk factors. In particular, risk factors such as sex and age, smoking, total cholesterol level above a limit such as 250 mg/dl, family history, diabetes, target organ damage TOD, associated clinical conditions ACC or low plasma renin activity PRA may be entered. In context with the present invention TOD (target organ damage) and ACC (associated clinical conditions) are used in accordance with the definitions given in "2003 European Society of Hypertension - European Society of Cardiologic Guidelines for the Management of Arterial Hypertension, Page 1015". Low renin will be considered to be a decision parameter if a biochemical renin test result is below 1 ng/mL/HR (for the upright position of a patient and a three-day normal sodium diet). Renin can be tested by a renin blood test or a plasma renin activity (PRA) test.

In a risk factor display area 35, there will be an indication whether the specific risk factors mentioned above have been confirmed by the user by applying a confirmation button of the user input interface 32.

The display of the device 1 according to Figure 5 is made somewhat different from the display of the device according to Figure 1. In addition, a risk level display 37 is provided which indicates whether the risk of the patient to suffer from a cardiovascular disease in a certain period is low, moderate, high or very high.

In a driving factor display 36, it is indicated whether hypertension of the individual rather is kidney driven (see Figure 5) or brain driven (not shown in Figure 5). The number of blood pressure pulses considered for heart rate variability measurements are not displayed in the embodiment according to Figure 5.

In addition, the system 1 is provided with a decision making means which, on the basis of the determined data determines measures for treatment of prevention against hypertension and cardiovascular diseases. Such measures are displayed in a display area 41. Typical measures might be "begin drug treatment" as shown in Figure 5. Determination of blood pressure and heart rate variability in the system according to Figure 5 is made in the same manner as explained with reference to Figure 1 and 2.

Figure 6 shows a fourth embodiment of the invention. The system according to Figure 6 is substantially identical to the system as shown in Figure 5 with the only exception that heart rate variability is determined with an electrocardiographic measuring unit 21 instead of blood pressure pulses. Determination of heart rate variability is made identically as explained with reference to Figures 3 and 4.

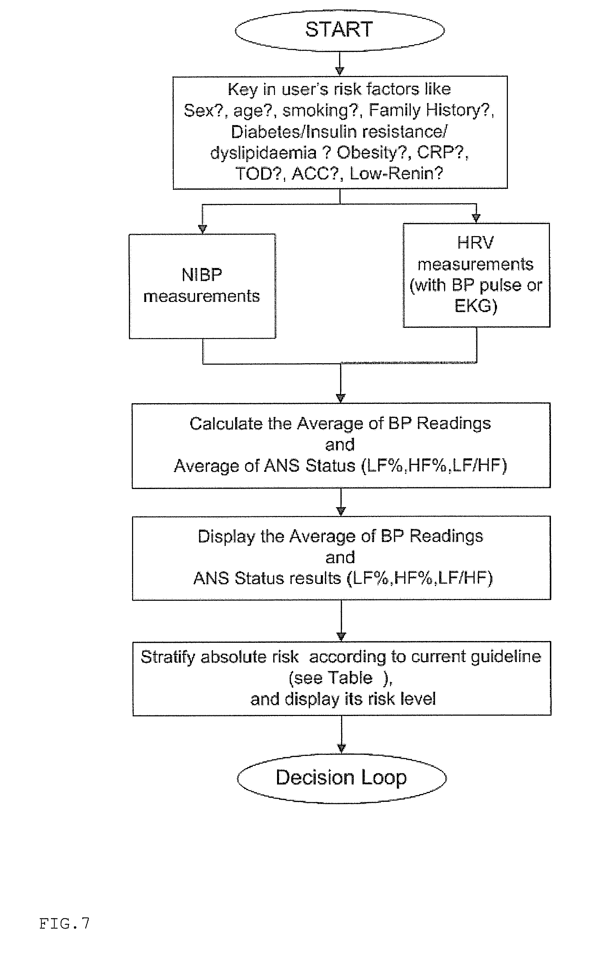

Figure 7 shows a flow chart for operation of a device 1 according to Figure 5 and 6. Before starting the measurement, users are required to enter risk factors such as sex, age, smoking, total cholesterol, family history, diabetes, target organ damage, associated clinical conditions and low renin. If diabetes relevant information also shall be considered, it is possible to enter also information relating to insulin resistance or dyslipidaemia. In a next step, non invasive blood pressure measurements are done. Heart rate variability is determined either by determining blood pressure pulses or by acquiring electrocardiographic information as explained with reference to Figure 2 or with reference to Figure 4.

After completion of these measurements, an average of blood pressure readings and an average of the autonomic nervous system status ANS, in particular of the low frequency rate LF, the high frequency rate HF and the ratio LF/HF is calculated. These results are displayed in a subsequent step.

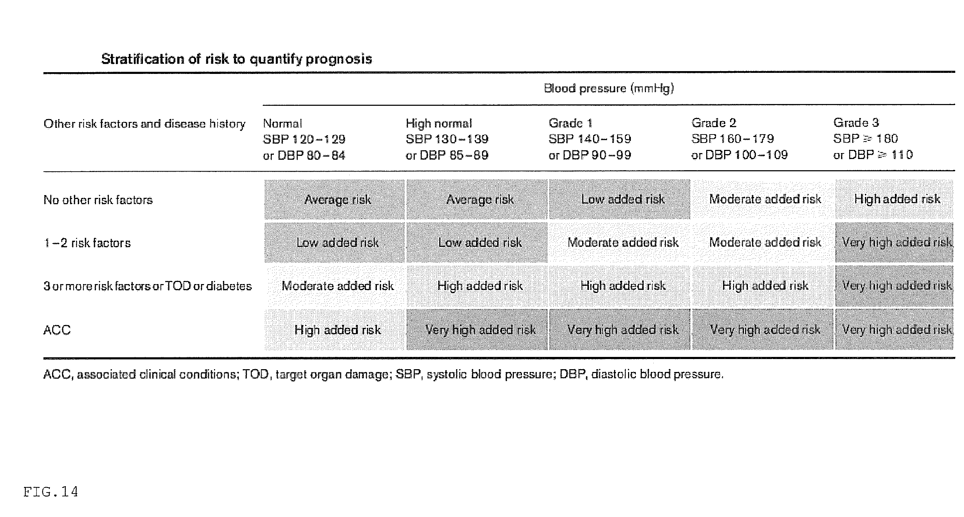

Based on these readings and on the risk factors, the risk level for the individual is stratified according to guidelines as shown in Figure 14. A risk level is displayed in the display 37.

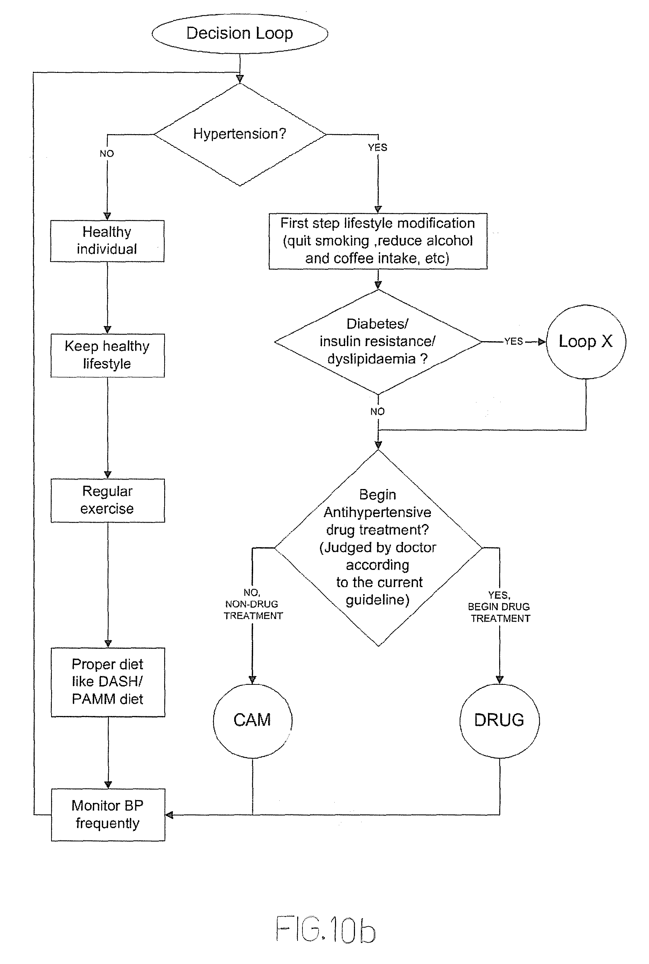

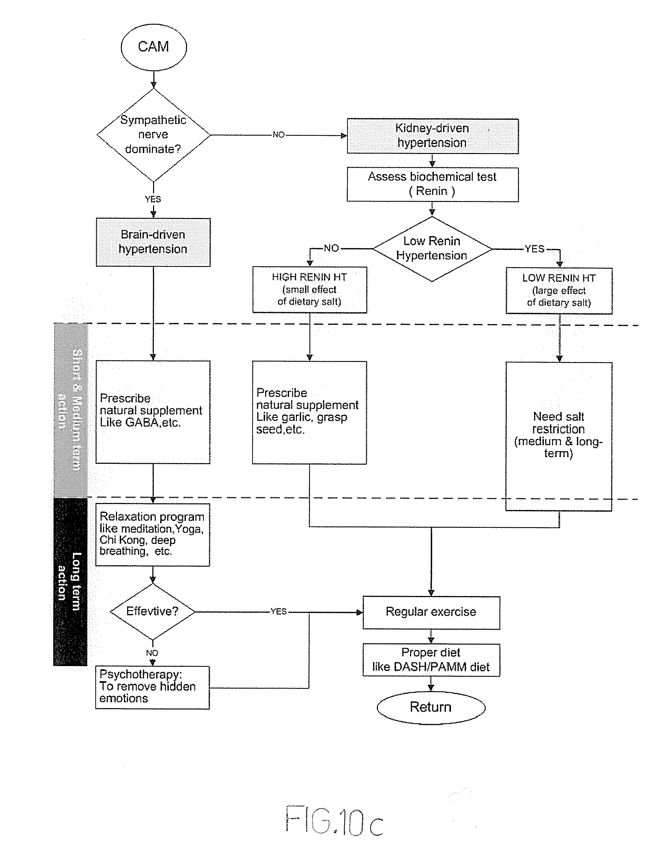

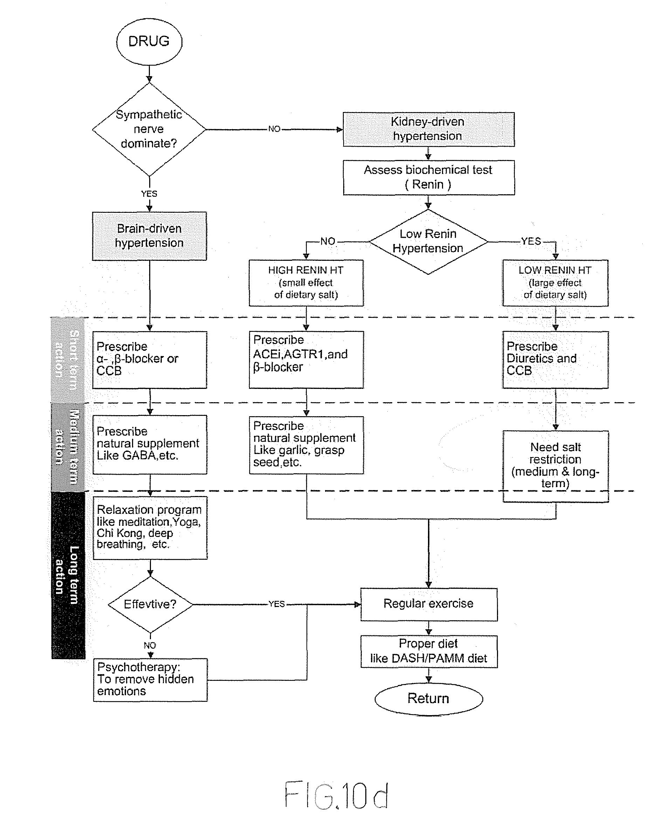

Thereafter, in a decision loop, the device starts to determine measures to be taken in view of treatment of the patient in the decision making means as will be explained hereinafter with reference to Figure 10b, 10c and 10d.

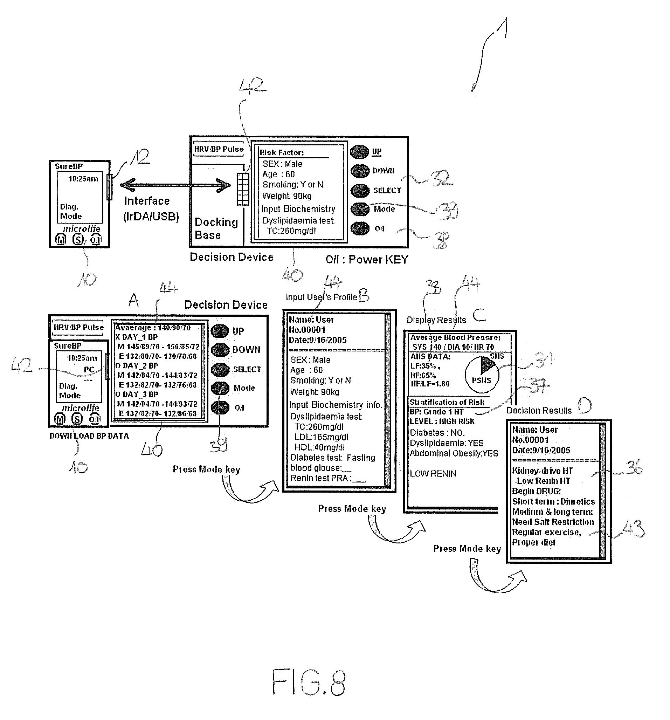

Figure 8 shows a fifth embodiment of a system 1 according to the present invention. The system 1 according to Figure 1 comprises a blood pressure measuring device 10 which is built as a stand alone system. The blood pressure measuring device is a diagnostic blood pressure measuring device which determines blood pressure according to a clinically validated, diagnostic measurement schedule. The blood pressure measuring device 10 is provided with a wireless communication interface 12 for exchange of data with a decision making device 40. The communication interface typically is an infrared IrDA interface. It is, however, also possible to use wire communications such as USB connections.

The decision device 40 is provided with a communication interface 42 for receiving data from the blood pressure measuring device. In the embodiment according to Figure 8, a user input interface 32 is provided in the decision making device 40. Risk factors and decision parameters which can be entered in the user input interface are similar or identical to the risk factors disclosed in context with the embodiment of Figure 5 and 6.

An operation interface 38 is provided with buttons for operation of the device. In the lower part of Figure 8, several screens which may be indicated in the display screen 44 of the decision device 40 are shown. In the initial status A of the screen 44, measurement results such as averages of blood pressure measurements on specific days are indicated. These data are acquired from the blood pressure measuring device 10 which is coupled to the device 40. By pressing a mode key 39 the display in the device is switched from status A to status B. In status B, the screen 44 allows to enter/verify risk factors in a similar manner as explained with reference to Figure 5 and 6. Biochemistry information may also be entered. By pressing mode key 39 a further time, the screen 44 switches to status C. In status C, heart rate variability data are shown in the heart rate variability display section 33. Risk levels are displayed in the risk level display 37 A doctor can input the result of renin tests determined in a biochemical test, e.g. in a patient's blood serum. By further pressing of mode key button 39, the screen 44 is switched to status D. In a driving factor display area 36, there is a display of the driving factor for hypertension, in the embodiment of Figure 8 kidney and low renin driven hypertension. In a display section 43 for measures to be taken, measures for treatment are displayed. This may e.g. be short term measures such as diuretics and medium and long term measures such as salt restriction/regular exercise/proper diet shown in Figure 8 by way of example.

Heart rate variability is determined on the basis of blood pressure pulses as has been disclosed herein above.

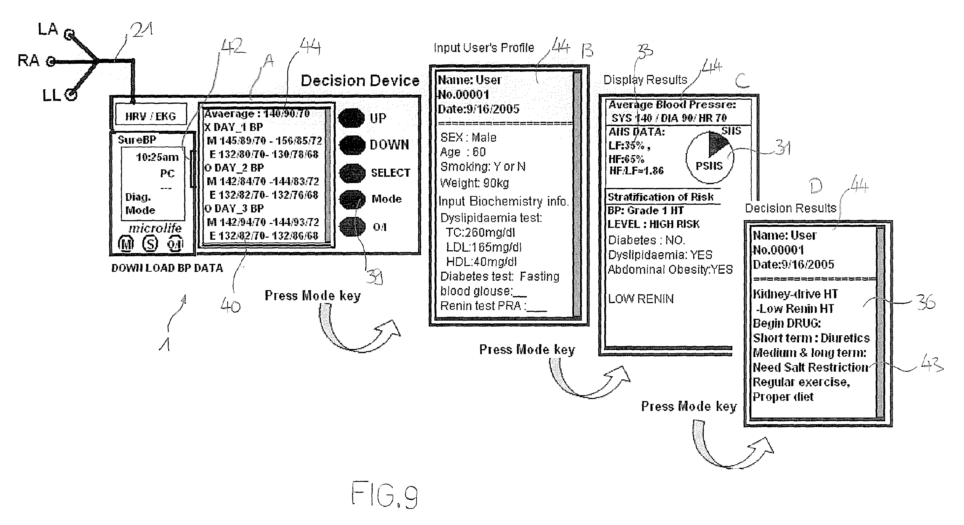

Figure 9 shows a sixth embodiment of the invention. The embodiment of Figure 9 is substantially identical to the embodiment of Figure 8. Contrary to the embodiment of Figure 8, according to Figure 9, heart rate variability is measured with a electrocardiographic unit 21 as disclosed in context with Figure 3 or 6. Operation of the device is otherwise identical to operation of the device as explained with reference to Figure 8.

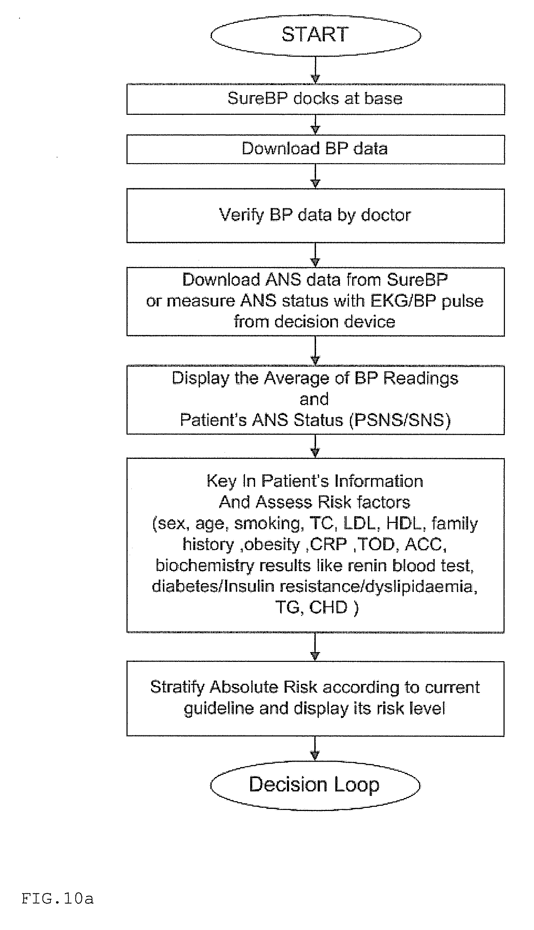

Operation of the devices according to the embodiments of Figure 8 and 9 will now be shown with reference to the flow chart of Figures 10a to 10d. In a first step (not shown in Figure 10a) blood pressure measurements are made in the blood pressure measuring monitor 10 in accordance with the disclosure with reference to Figure 7. Determined averages are displayed and stored within the blood pressure monitor 10. The blood pressure monitor 10 (SureBP) is then docked at a docking base of the decision making device 40. Upon docking, blood pressure data are automatically downloaded to the decision making device 40. The blood pressure data are subsequently verified by a care person or a doctor in view of their reliability. In particular, it may be verified whether measurements had been made on a working day of the individual. The display 44 may also display the date associated with each recorded data. Thus, a doctor or a care person can judge whether measurements have been acquired on a working day or on a non-working day. Measurement values can be edited (i.e. removed) by means of display menus and up/down and selection operation keys.

In a further step, autonomic nervous system data ANS are downloaded from the blood pressure measuring device 10 in accordance with the embodiment of Figure 8. In the embodiment of Figure 9, autonomic nervous system data are acquired with a electrocardiographic determination system which may be integrated in the decision making device 40.

It is, however, also possible to have a separate blood pressure pulse based heart rate variability determination means integrated within decision making device 40.

The averages of the blood pressure readings and the autonomic nervous system activity status will then be determined and displayed on the autonomic nervous system activity status display 31 (see Figure 8 or 9).

In the next step, the user or doctor is asked to enter information relating to the risk factors. Risks for the individual to suffer from cardiovascular diseases are then stratified and displayed as a risk level in the risk level display 37 (see Figure 8 and 9). Stratification is done in accordance with the guidelines as shown with reference to Figure 14.

Appropriate measures for treatment/prevention will then be determined in a decision loop which will be explained with reference to Figure 10b. In a first step, it is decided whether the individual suffers from hypertension. If the individual does not suffer from hypertension, it is considered that the individual is healthy. There will be a display indicating "healthy individual". Appropriate measures will then be displayed. Typically, appropriate measures or displays for healthy individuals may be "keep a healthy lifestyle", "practice regular exercise", "have a proper diet" (such as dietary approach to stop hypertension (DASH) and/or Pan-Asian Modified Mediterranean diet PAMM). Finally, the individual is asked to monitor blood pressure frequently by display of a message "monitor BP frequently".

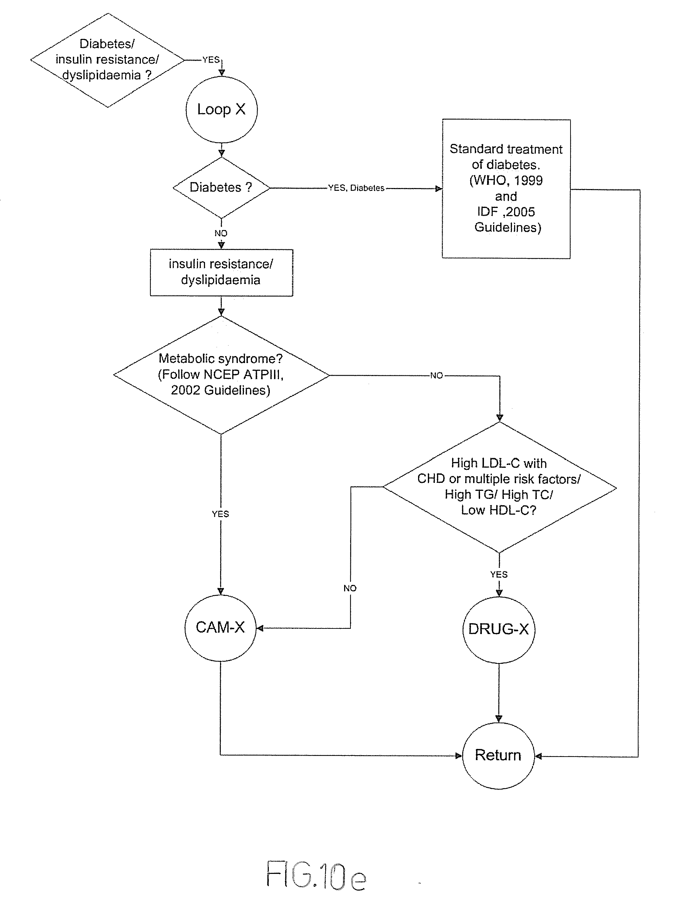

If the individual is found to suffer from hypertension, in a first step lifestyle modifications such as quit smoking, reduce alcohol and coffee intake, reduce risk factors such as high total cholesterol or obesity will be displayed. In a further step, it is checked whether the individual suffers from diabetes or has insulin resistance or dyslipidaemia. This judgement which may be optional is based on risk factors and biochemistry test data entered into the device as explained with reference to the flow chart in Figure 10a. If the answer to this test is "yes", in a separate loop (see Figure 10e) specific, diabetes related determination routine will be made. If the answer is "no", a judgement as to whether a drug treatment shall be started is done by a doctor according to current guidelines.

This judgement is based on criteria as outlined in Figure 1 on page 1024 of "2003 European Society of Hypertension/European Society of Cardiology Guidelines for the Management of Arterial Hypertension".

If no drug treatment shall be started, in a complementary and alternative medicine mode CAM (see Figure 10c) the device determines appropriate measures. If a drug treatment shall be started, the device determines appropriate medication in a drug mode DRUG as explained with reference to Figure 10d.

Figure 10c shows a complementary alternative medicine determination mode. In a first step, it is judged whether the sympathetic nervous system dominates in the individual. If the autonomic nervous system activity status HF/LF as determined and displayed earlier is above 1/1, it is considered that the asympathetic nervous system dominates. The decision device will prompt for an entry of an appropriate treatment by the doctor. According to an alternative embodiment, it is also possible for a doctor to switch between a complementary and alternative medicine mode and a drug treatment mode by manually changing the operation. If the sympathetic nervous system is found to dominate, the patient is considered to suffer from brain driven hypertension. In a next step, as short and medium term actions, natural supplements are prescribed. Such natural supplements typically may be Gamma aminobutyric acid or other appropriate supplements. As long term actions, relaxation programs such as mediation, yoga, Chi Kong, deep breathing or other measures will be prescribed.

If these measures have been effective, regular exercise and proper diet will be suggested to the individual. If these long term actions are not effective, psychotherapy in view of removal of hidden emotions may be prescribed.

The system according to the invention continues to monitor the user's hypertension until hypertension is found to be sufficiently well controlled. Measurements may be repeated, e.g. on a daily basis.

If the sympathetic nervous system does not dominate, the individual is considered to suffer from kidney driven hypertension.

In a further step, biochemical test results, in particular renin level in the blood volume are assessed. Information relating to renin level has been entered as a decision parameter see Figure 7). If renin is high, i.e. if renin is above 6 ng/mL/Hr in a PRA test, the patient is considered to suffer from high renin hypertension. In this case, natural supplements such as garlic tension. In this case, natural supplements such as garlic or grasp seeds are prescribed. If the patient is considered to suffer from low renin hypertension, i.e. if renin is below 1 ng/mL/Hr, salt will strongly effect hypertension. In this case, salt restriction is displayed as a medium and long term measure.

Finally, regular exercise and proper diet is suggested in both cases to the patient.

Figure 10d shows a drug determination mode. Distinction between brain driven, kidney driven and high or low renin driven hypertension is made in a similar manner as described with reference to Figure 10c.

For brain driven hypertension, alpha-, beta- or CC-blockers are prescribed as a short term action. Medium term and long term actions prescribed are identical to the actions as disclosed with reference to Figure 10c.

If the patient is found to suffer from kidney driven high renin hypertension, medication such as ACEi, AGTR1 or beta-blockers are prescribed. If the individual is considered to suffer from low renin hypertension, diuretics and CCB are prescribed. Medium and long term actions are the same as explained with reference to Figure 10c.

If in the judgement step related to diabetes shown in Figure 10b the answer is "yes", a diabetes related decision loop as shown in Figure 10e will be started. If the patient is found to suffer from diabetes, a standard treatment of diabetes according to the World Health Organisation 1999 and IDF 2005 Guidelines will be suggested and further determination in view of antihypertensive treatment as shown in Figure 10b will be continued. If the patient is not found to suffer from diabetes, it is assumed that the patient either has insulin resistance or dyslipidaemia.

Typically, insulin resistance will be deemed to be present if fasting glucose is between 110 and 125mg/dl. Boradline dyslipidaemia is deemed to be present if TC is between 200 and 239 mg/dl, LDL-c is 130 to 159 mg/dl, TG is 150 to 199 mg/dl and HDL is <40 mg/dl. In a further step, it is checked whether the patient suffers from a metabolic syndrome as defined in the NCEP ATPIII 2002 Guidelines.

A doctor or a user can input biochemistry test results relating to risk factors or related biochemistry test data such as fasting blood glucose, TC, TG, LDL, HDL or other results. According to the above mentioned guidelines, a determination if patients suffer from insulin resistance or dyslipideamia may be made in the decision device. The result of this determination may be displayed in the device.

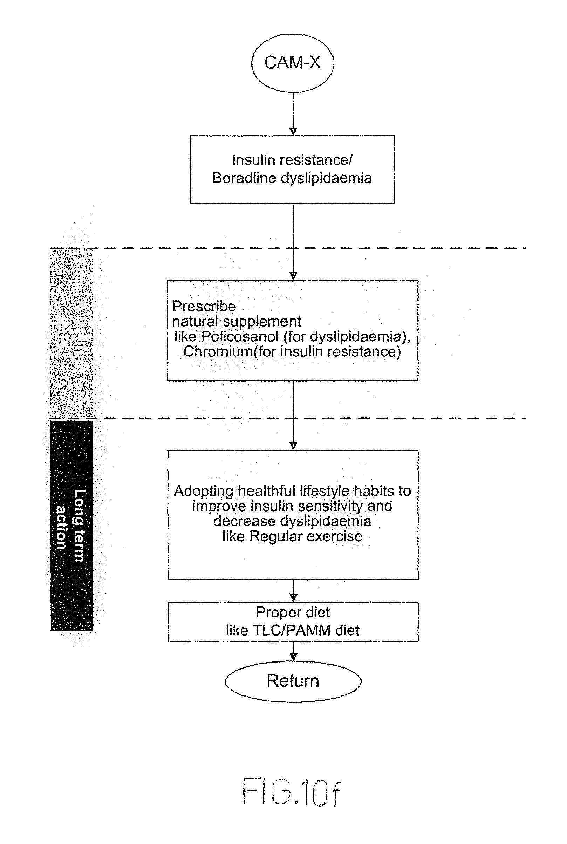

If the patient is found to suffer from the metabolic syndrome, a complementary alternative medicine treatment determination sequence as shown in Figure 10f will be started. Otherwise, in a next step it will be checked whether the patient has risk factors such as high LDL-C with CHD (coronary heart disease) or whether the patient suffers from high LDL-C and high TG/high TC and low HDL-C. This decision is based on patient's information as keyed according to the flow chart shown in Figure 10a.

If the answer to the judgement is "no", the complementary alternative medicine routine as shown in Figure 10f will be started. If the answer is "yes", a drug treatment routine as shown in Figure 10g will be started. After the treatment routines, which will be explained with reference to Figure 10f or 10g, the system returns to the antihypertensive treatment as shown in tem returns to the antihypertensive treatment as shown in Figure 10b.

According to the NCEP ATP III 2002 Guidelines typically, diagnostic criteria for the "metabolic syndrome" may be a waist conference of >102cm (for male) or >88cm (for female), fasting glucose >110mg/dl, HDL-C <40mg/dl (for male) or <50mg/dl (for female), TG >=150mg/dl or an arterial pressure >=130/85mm HG. Typically, metabolic syndrome will be considered to be present if there are more than three risk factors as mentioned above.

Figure 10f shows typical short and medium term actions and long term actions for treating a patient suffering from insulin resistance or dyslipidaemia with complementary alternative medicine. Typically, as a short and medium term action, natural supplements like Poliocosanol (for dyslipidaemia) or Chromium (for insulin resistance) are prescribed. As a long term action, typically life style change actions will be proposed.

Figure 10g shows a routine relating to a drug treatment in view of high LDL-C with CHD or high LDL with multiple risk factors. This routine will be applied if LDL-C is above 130 mg/dl with CHD or if LDL is above 160 mg/dl with multiple of the risk factors TG > 200 mg/dl, TC > 240 mg/dl or HDL-C < 40 mg/dl.

As a short term action, medication such as stains, cholestyramine, niaspan, clofribrate or the like are prescribed. As a medium term action, natural supplements such as poliocosanol may be prescribed. As a long term action, appropriate life style changes will be suggested.

Prescriptions determined in accordance with the rules of Figure 10c and 10d or 10f and 10g are displayed in status D of the display 41 as indicated in Figure 8 and Figure 9.

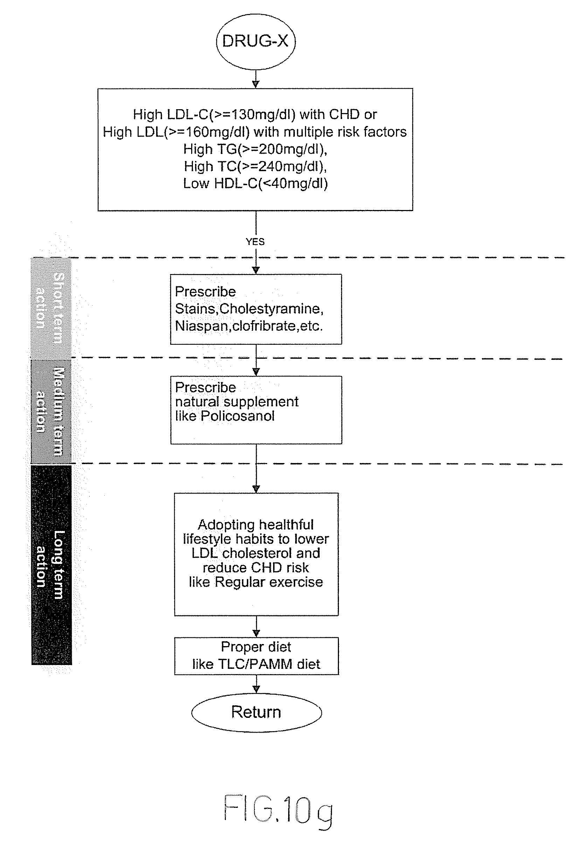

Figure 11 shows a further embodiment of a system 1 in accordance with the invention. A blood pressure measuring device 10 and a heart rate variability measurement device 21 based on electrocardiographic information are acquiring blood pressure and heart rate variability data as disclosed herein above. These data are transferred with wire or wireless communication or also by manual entry into a personal computer or personal digital assistant 50. The personal computer or personal digital assistant 50 is provided with a software and input allowing operation identical to operation of the stand-alone decision making device 40 described in Figure 8 and 9.

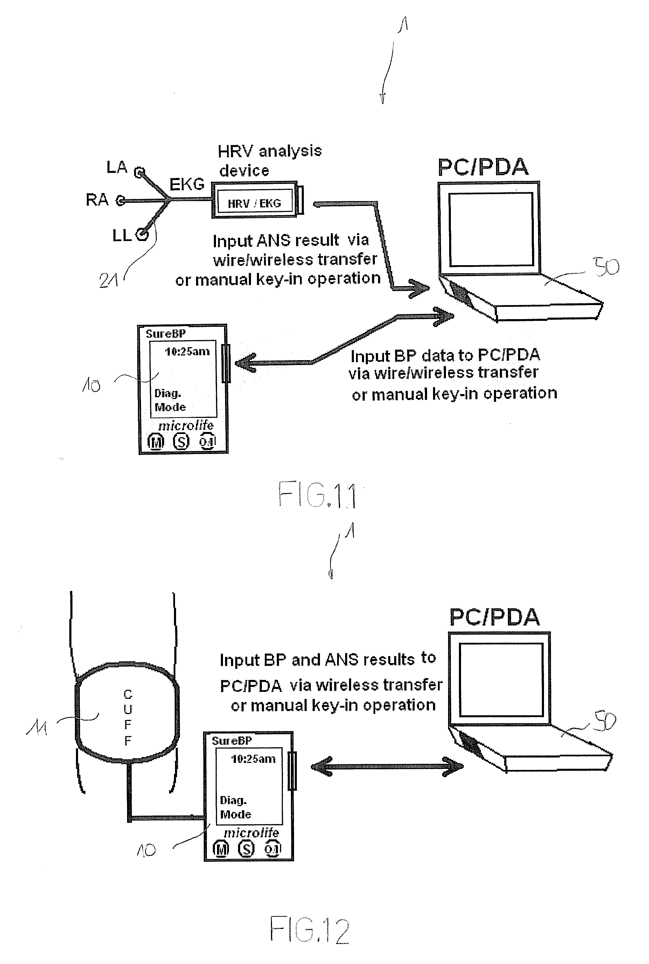

Figure 12 shows a further alternative embodiment. The embodiment of Figure 12 is identical to the embodiment of Figure 11 with the exception that the blood pressure measuring device 10 is designed to determine heart rate variability in accordance with the system disclosed in Figure 1.

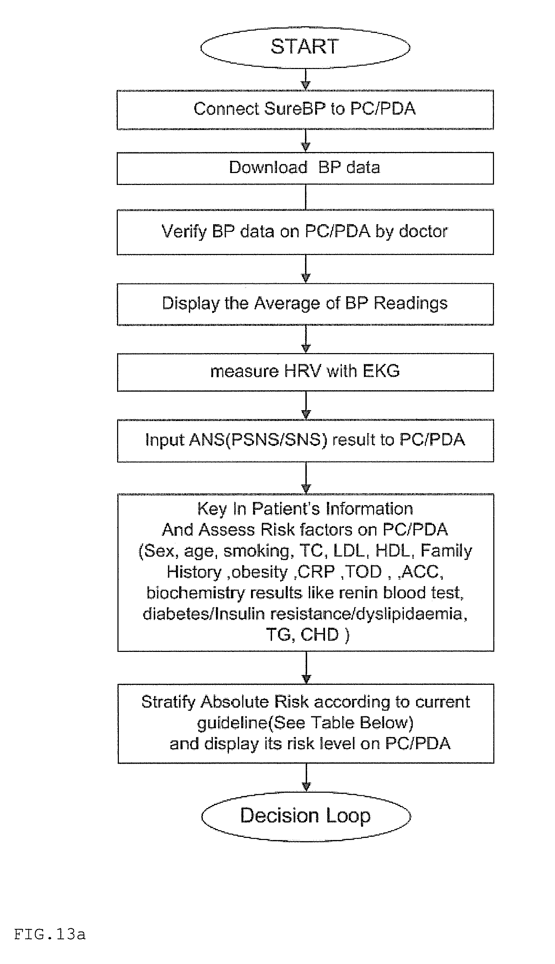

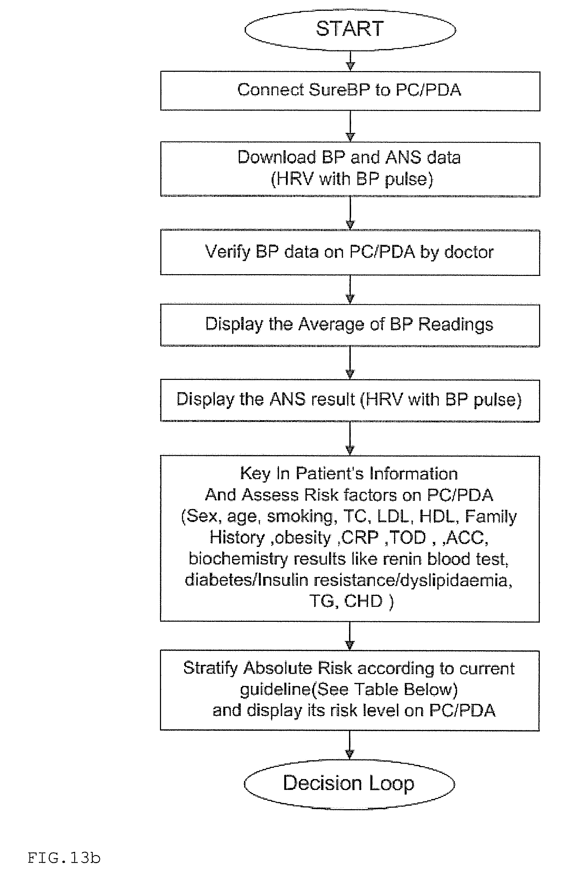

A flow chart of operation of the devices of Figure 11 and 12 is shown in Figure 13a and a flowchart of operation of the device according to Fig. 12 is shown in Fig. 13b. The flow chart of Figure 13a and Fig. 13b is substantially identical to the flow chart of Figure 10a. The only difference is that the blood pressure monitor 10 is connected to a personal computer/persona digital assistant instead of connection to a separate decision making device.

Figure 14 by way of example shows a table for risk stratification. This table is based on suggestions of the World Health Organisation. Typically, depending on the number of risk factors, on presence of specific risk factors such as target organ damage, diabetes or associated clinical conditions, different risks such as low, moderate, high and very high risks are determined on the basis of blood pressure measurements.

Figure 16a shows the heart rate variability power spectrum as e.g. shown in "heart rate variability, standards of measurement, physiological interpretation, and clinical use, circulation 1996/93: 1043 - 1065". The HRV power spectrum can be divided into four components based on the frequency range. The ultra low frequency (ULF) is a frequency having a power density number below 0,003 Hz. The very low frequency range (VLF) has a power density number between 0,003 and 0,04 Hz. The low frequency (LF) has a power density number between 0,04 and 0,15 Hz. Such frequency range is mainly generated by sympathetic nervous activity. The high frequency range (HF) corresponds to power density numbers between 0,15 and 0,4 Hz. High frequency is derived from the vagal activity. This activity is modulated by respiration.

Since LF represents mainly sympathetic activity and HF represents vagal activity, the ratio HF/LF is a good indicator of the autonomic nervous balance.

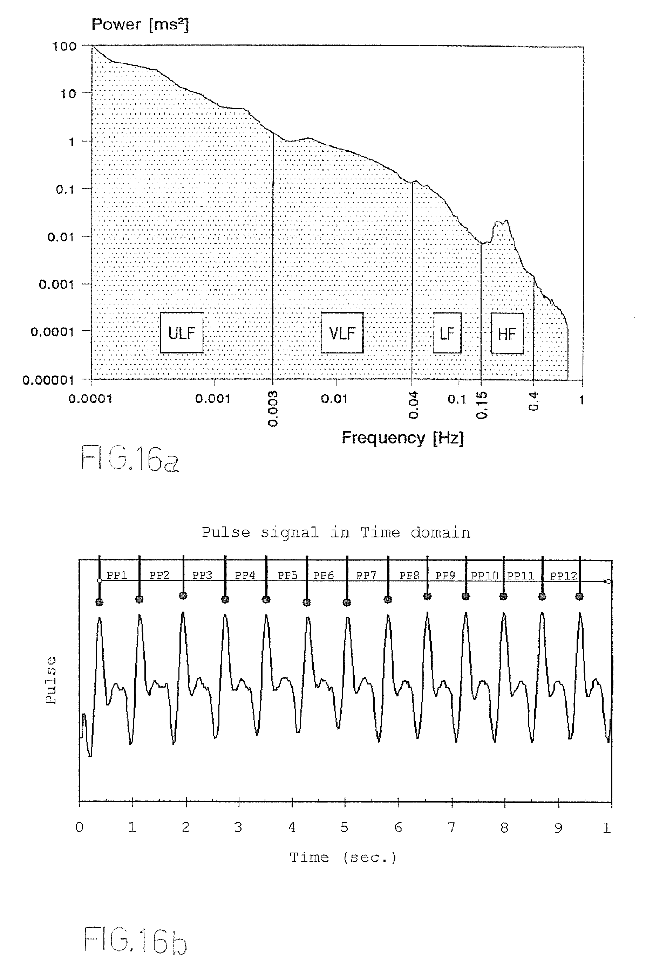

Figure 15 shows a flow chart for determining heart rate variability. In a first step, heart rate variability information will be sampled. This will be done by making a measurement based on electrocardiographic signals or blood pressure pulse signals. Sampling is typically made with a rate of 128 Hz. On the basis of sampled data, peaks and interval times between subsequent peaks will be measured. Such pulse time intervals PP1, PP2, ... typically are shown in Figure 16b.

In a further step, abnormal pulses which are deemed to be based on artefacts will be rejected. Methods for artefact determination are known to those skilled in the art.

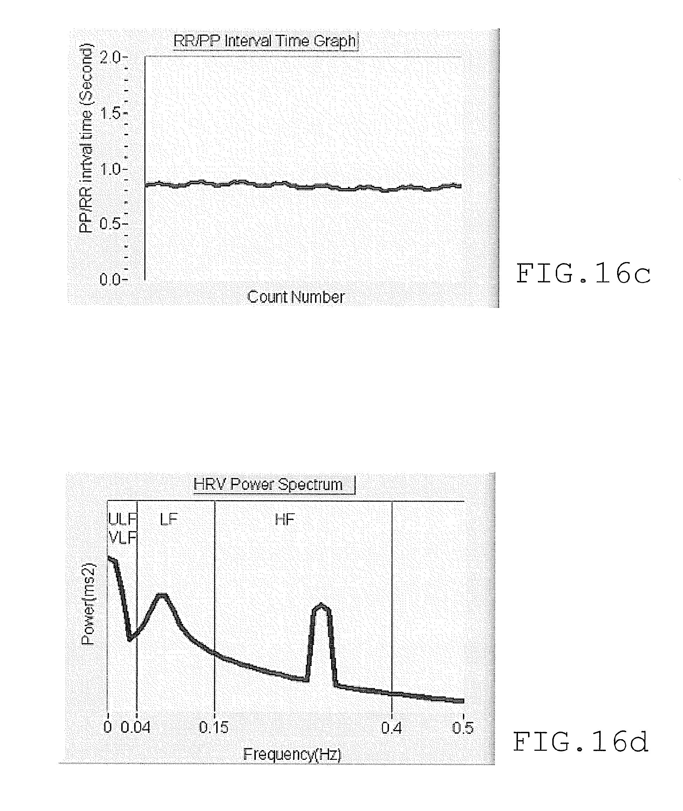

In a further step as shown in Figure 16c, each valid interval time (PPN for pressure pulses or RRN for intervals time between R-waves of subsequent electrocardiographic pulses) will be saved in a time domain. Such interval times in dependence of the number of heart beats (count number) are shown in Figure 16c.

In a further step power spectral density of HF and LF (see Fig. 16d) will be determined by Fast Fourier transformation on the PP or RR data in a manner known to those skilled in the art. A power spectral analysis of the interval variability in a specific frequency domain will be made.

Such a heart rate variability power spectrum is shown with reference to Figure 16d. The low frequency component is considered to be the power density number for the low frequency range (range between 0,04 and 0,15 Hz). The high frequency component is the power density number for the high frequency range between 0,15 and 0,40 Hz. With a normalisation procedure by dividing the power density of the high frequency range and the power density of the low frequency range a percentage will be determined which can be displayed in the bar graph 31 (shown e.g. in Figure 1).

HVR thus is basically determined in accordance with the teaching of "heart rate variability, standards of measurement, physiological interpretation, and clinical use, circulation 1996/93: 1043 - 1065", which is incorporated by reference into the present application.

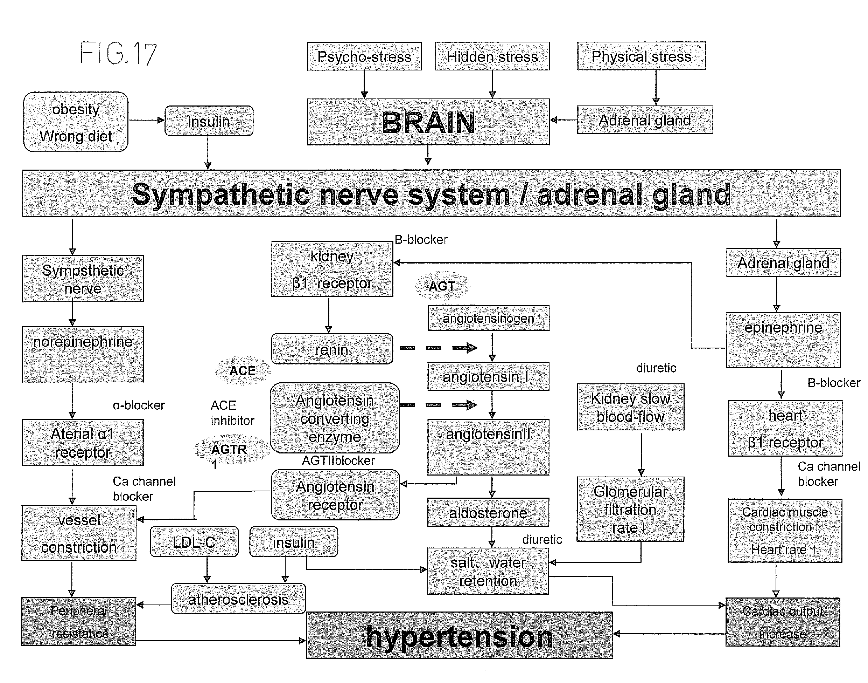

Figure 17 shows a map explaining processes in context with function and route causes of hypertension and related diseases.

The left and the right hand side branch of figure 17 show pathways leading to hypertension which are based on influences from the brain to the sympathetic nervous system (left branch) and to the adrenal gland (right branch). Where and how medication such as alpha blockers, beta blockers or Ca channel blockers intervene in this pathway is indicated.

Hypertension as a result of these pathways has its roots mainly in psycho stress, hidden stress or physical stress. Such hypertension is rather brain driven. A wrong diet and obesity may furthermore influence these pathways.

The middle branch of figure 17 shows a kidney driven pathway which influences hypertension, e.g. by intake of too high amounts of salt. The genetic effect of e.g. AGT, ACE or AGTR1 or Renin on production or conversion of angiotensin I and II and aldosterone is shown. Counter measures by medication such as ACE inhibitors or AGTII blockers are shown and may be explained to the individual on the basis of this map.

Figure 17 forms the basis for determining medications suggested in accordance with the method and system disclosed herein above.

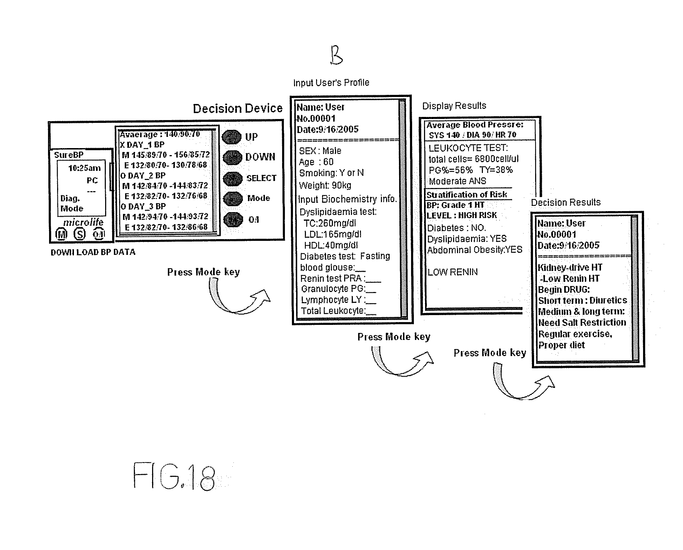

Figures 18 to 20 show further embodiments of the invention which are based on a different determination of the activity of the nervous system. It is known that the activity of the autonomic nervous system also may be assessed on the basis of the composition ratio of granulocytes and lymphocytes in leukocytes. Such procedure has been proposed by Toru Abo in "Immunomodulation by the autonomic nervous system" in recent Res.Devel.Immunology, 4 (2002): 559-578. The embodiment shown in Figure 18 is substantially similar to the embodiment e.g. shown in Figure 8. However, instead of determination by means of blood pressure pulse measurements, the activity of the autonomous nervous system is based on results of leukocyte tests. These test results are entered into the device in screen B as shown in Figure 18.

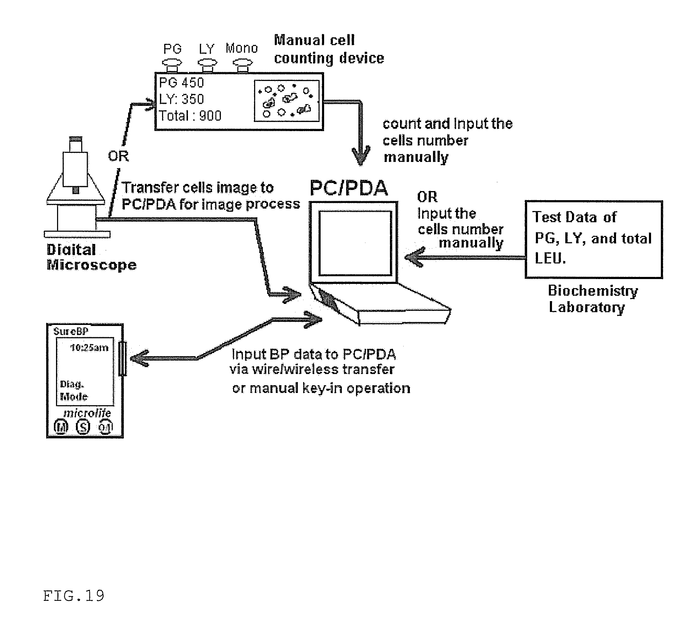

Figure 19 shows a further embodiment where the activity of the nervous system is determined based on leukocyte test results. Blood pressure values are transferred to a personal computer or personal digital assistant in a similar way as shown with reference to Figure 11 and Figure 12. However, based on a leukocyte test, results taken in a digital microscope are either transferred as cell images to a personal computer/personal digital assistant for further image processing or are transferred to a manual cell counting device in order to determine the composition ratio between PG (amount of granulocytes) and LY (amount of lymphocytes).

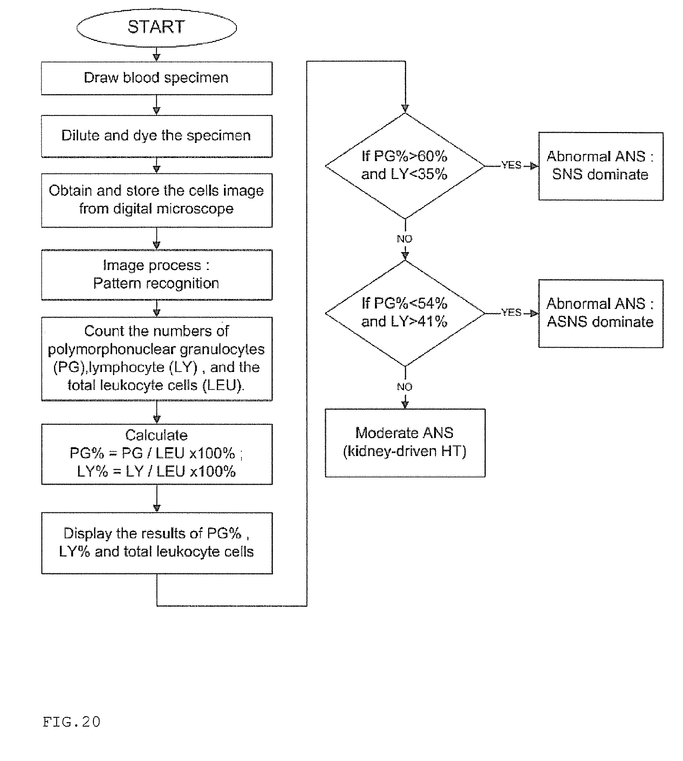

In Figure 20 a flow chart of such a process is shown. In the left branch of Figure 20, the operation steps of blood sample taking are shown. A sample of blood is drawn, diluted and dyed. Cell images are obtained and stored from a digital microscope. By means of image processing, pattern recognition is done and the number of polymorphonuclear granulocytes (PG), lymphocytes (LY) and the total leukocyte cells (LEU) are counted. A PG percentage PG% and a LY percentage LY% is calculated as a ratio of LEU. The results of PG% and LY% and the total leukocyte cells are displayed. If PG is >60% and LY <35% it is considered that the autonomic nervous system is abnormal and that the sympathetic nervous system dominates. If PG <54% and LY >41% it is deemed that the autonomic nervous system is abnormal and that asympathetic nervous system ASNS dominates.

If PG is above 54% and below 60% and LY is between 35% and 41% it is deemed that the autonomic nervous system is moderate and that the individual may suffer from kidney driven hypertension.

## Claims

1. A system (1) for the management or control of cardiovascular related diseases, in particular of hypertension of an individual, comprising
- a blood pressure measuring device (10) for measuring the blood pressure of the individual
- means for determining an autonomic nervous system activity status of said individual.

2. A system according to claim 1, the system further comprising a heart rate variability determination means (20) for determination of the heart rate variability (HRV) of said individual.

3. A system according to claim 1, the system further comprising means for determining or entering a composition ratio of granulocytes and lymphocytes in leukocytes of the individual.

4. A system according to claim 2, further comprising a calculating means for determining an autonomic nervous system activity status based on said heart rate variability (HRV).

5. A system according to claim 1 to 4, wherein said system (1) comprises a display for display of said autonomic nervous system activity status.

6. A system according to one of the claims 1 to 5, wherein said blood pressure measuring device (10) and said heart rate variability (HRV) determination means (20) are integrated within the same physical device (30).

7. A system according to one of the claims 1 to 6, wherein said heart rate variability determination means (20) is adapted to determine heart rate variability (HRV) on the basis of blood pressure pulse measurements.

8. A system according to one of the claims 1 to 6, wherein the heart rate variability determination means (20) include an ECG measuring means (21) and wherein said heart rate variability means (20) is adapted to determine heart rate variability (HRV) on the basis of electrocardiographic information of said individual.

9. A system according to one of the claims 1 to 8, wherein said system (1) comprises a user input interface adapted for entry of information relating to at least one of risk factors of said individual to suffer from diseases, in particular from cardiovascular related diseases and/or driving factors for such disease.

10. A system according to claim 9, wherein said user input interface is adapted for entry of information selected from the group of sex, age, smoking, total cholesterol level, family history, target organ damage, diabetes, associated clinical conditions and renin concentration or other biochemical information.

11. A system according to one of the claims 1 to 10, wherein said blood pressure measuring device (10) is designed for measurement of blood pressure according to a predetermined or predeterminable clinically validated measurement schedule.

12. A system according to one of the claims 1 to 11,
wherein said system (1) comprises a decision making means (40) coupled or adapted to be coupled to said blood pressure measuring device (10) and to said autonomic nervous system activity status determination means and wherein said decision making means comprises data processing means for determining at least one of a risk level for the patient to suffer from a disease and/or driving factors for such disease and/or appropriate measures for treatment of the individual in view of said disease on the basis of said blood pressure measurement and autonomic nervous system activity status determination.

13. A system according to claim 12, wherein said data processing means are adapted to make medication or treatment suggestions based on data acquired by the autonomic nervous system activity status determination means, on data acquired by the blood pressure measuring device (10) and on data entered by the user or care person, in particular based on the fact whether hypertension has been found to be brain driven or kidney driven.

14. A system according to one of the claims 12 to 13, wherein said decision making device comprises display means for display of at least one of short, medium and long term measures in view of treatment of said individual.

15. A system according to one of the claims 11 to 14, wherein said decision making means is a separate device and wherein the decision making means is provided with a wire or wireless communication or manual entry interface for communication with said blood pressure measuring device (10) and/or said autonomic nervous system activity status determination means.

16. A system according to one of the claims 1 to 15, wherein said system (1) comprises follow up means for determining the effectiveness of a treatment or compliance of an individual with a prescribed measurement, treatment or medication schedule.

17. A method for operating a system (1) for the management or control of cardiovascular related diseases, in particular of hypertension of an individual, in particular in accordance with one of the claims 1 to 16, comprising the steps of
- acquiring blood pressure data of said individual
- determining an autonomic nervous system activity status of said individual
- based on said autonomic nervous system activity status and on said blood pressure data, determining a reason for hypertension or a risk level of the disease and/or defining short, medium and/or long term measures in view of treatment or prevention of said individual against cardiovascular related diseases, in particular hypertension.

18. A method according to claim 17, comprising the step of acquiring heart rate variability data (HRV) of said individual and determining an autonomic nervous system activity status based on said heart rate variability (HRV) data.

19. A method according to claim 17, comprising the step of acquiring a composition ratio of percentages of granulocytes and lymphocytes in leukocytes of said individual and determining an autonomic nervous system activity status based on said ratio.

20. A method according to claim 17, comprising the further step of displaying said autonomic nervous system activity status on a display.

21. A method according to one of the claims 17 or 18, wherein said heart rate variability (HRV) is determined on the basis of blood pressure pulse measurements.

22. A method according to one of the claims 18, wherein said heart rate variability (HRV) is determined on the basis of electrocardiographic information.

23. A method according to one of the claims 17 to 22, comprising the further step of acquiring information relating to risk factors and biochemical information for the individual to suffer from a cardiovascular related disease, in particular from hypertension.

24. A method according to one of the claims 17 to 23, wherein said blood pressure measurements are made in accordance with a predetermined or predeterminable clinically validated measurement schedule.

25. A method according to one of the claims 17 to 24, comprising the further step of display of measures in view of treatment or prevention of disease of said individual on a display.
